# EUROPÄISCHE PATENTSCHRIFT

(11) **EP 1 458 426 B1**
(45) Veröffentlichungstag und Bekanntmachung des Hinweises auf die Patenterteilung: **18.08.2010**
(21) Anmeldenummer: 02798275.0
(22) Anmeldetag: 13.12.2002
(51) Int. Cl.: A61L 27/04

(54) **MEDIZINISCHE IMPLANTATE, PROTHESEN, PROTHESENTEILE, MEDIZINISCHE INSTRUMENTE, GERÄTE UND HILFSMITTEL AUS EINEM HALOGENID-MODIFIZIERTEN MAGNESIUMWERKSTOFF**
MEDICAL IMPLANTS, PROSTHESES, PROSTHESIS PARTS, MEDICAL INSTRUMENTS, DEVICES AND AUXILIARY CONTRIVANCES MADE OF A HALOGENIDE-MODIFIED MAGNESIUM SUBSTANCE
IMPLANTS MEDICAUX, PROTHESES, PARTIES DE PROTHESES, INSTRUMENTS MEDICAUX, APPAREILS ET ACCESSOIRES MEDICAUX CONSTITUES DE MATIERE DE MAGNESIUM MODIFIEE PAR HALOGENURES

(30) Priorität: 24.12.2001 DE 10163106
(43) Veröffentlichungstag der Anmeldung: 22.09.2004
(73) Patentinhaber: Universität Hannover, 30168 Hannover (DE); Medizinische Hochschule Hannover, 30625 Hannover (DE)
(72) Erfinder: WIRTH, Carl-Joachim, 30916 Isernhagen (DE); WINDHAGEN, Henning, 30159 Hannover (DE); WITTE, Frank, 30625 Hannover (DE); BACH, Friedrich-Wilhelm, 30916 Isernhagen (DE); KAESE, Volker, 30161 Hannover (DE)
(74) Vertreter: Kröncke, Rolf
(86) Internationale Anmeldenummer: PCT/DE2002/004568
(87) Internationale Veröffentlichungsnummer: WO 2003/055537

(56) Entgegenhaltungen:
- WO-A-99/03515
- DE-A- 1 953 241
- US-A- 5 405 428
- US-A- 5 571 188
- US-A1- 2008 255 045
- HAFERKAMP, HEINZ ET AL: "Alloy development, processing and applications in magnesium lithium alloys" MATERIALS TRANSACTIONS (2001), 42(7), 1160-1166, XP009009637
- DATABASE CA [Online] CHEMICAL ABSTRACTS SERVICE, COLUMBUS, OHIO, US; KAESE, VOLKER: "Contribution corrosion-preventing alloying of magnesium alloys" retrieved from STN Database accession no. 138:27790 XP002242085 & FORTSCHRITT-BERICHTE VDI, REIHE 5: GRUND- UND WERKSTOFFE/KUNSTSTOFFE (2002), 666, I-XII, 1-158 ,

## Beschreibung

Die Erfindung betrifft die Verwendung eines speziellen, in seiner Korrosivität veränderten Magnesiumwerkstoffs für die Herstellung medizinischer Implantate für den Einsatz im oder am menschlichen oder tierischen Körper, Protheseteile, Prothesen, medizinischer Instrumente, Geräte und Hilfsmittel, sowie diese Implantate, Prothesen, Instrumente, Geräte und Hilfsmittel selbst.

Die Erfindung bezieht sich im weiteren Sinne auf resorbierbare Implantate, die zur Freisetzung von Medikamenten oder als Befestigungselemente im Hart- und Weichgewebe Anwendung finden.

Resorbierbare Implantate werden bis dato durch Polymere dargestellt. Diese weisen jedoch zwei gravierende Nachteile auf: Zum einen werden bei der Freisetzung körperschädliche Weichmacher freigesetzt, zum anderen sind die mechanischen Eigenschaften der Polymere unbefriedigend.

Bereits seit Beginn des 20. Jahrhunderts ist bekannt, dass Implantate aus Magnesium und seinen Legierungen Vorteile mit sich bringen, da sie leicht resorbierbar und biokompatibel sind. Die Resorbierbarkeit im Körper erfolgt auf Grund der Korrosion von Magnesium in salinen Immersionen. Der essentielle Charakter für die Körperfunktionen und das Ausschwemmen von Überdosen über die Harnwege qualifizieren Magnesium als Implantatbasiswerkstoff mit hoher physiko-chemischer Biokompatibilität. Die durchschnittliche Verteilung in der Körpermasse liegt bei 470 mg/kg, die empfohlene Tagesdosis bei 200 bis 300 mg/d MgSO₄. Weiterhin wirkt Magnesium antiarrythmisch und senkt den Blutdruck sowie die Schmerzempfindlichkeit. Die maximale Dosis für eine Kurzinfusion in einen Menschen, Masse 75 kg, liegt bei 57,6 mg Reinmagnesium. Das Blutplasma enthält z.B. 107 mMol/l, der Magensaft 160 mval/l an Magnesiumchloridionen, so dass Magnesium in diesen salinen Immersionen korrodieren und so metallische sowie mechanisch belastbare aber biodegradable Temporärimplantate ermöglichen wird.

Die Entwicklungen der ersten Mg-Periode vor dem zweiten Weltkrieg (Verbrugge 1933, McBride 1938, Lambotte 1932) konnten keine Legierungen zur Verfügung stellen, die ausreichend langsam korrodieren und somit körperverträglich nur geringe Mengen Wasserstoff entwickeln. Die Entwicklungen der zweiten Mg-Periode während des kalten Krieges (Stroganov, DE-OS 1 953 241) brachten korrosionsfestere Legierungen, deren prinzipiell vorhandener Cadmium-Zuschlag das Knochenanwachsen beschleunigen sollte, aufgrund seiner Toxizität jedoch unter anthropologischen Aspekten in den westlichen Industrienationen nicht implantierbar wäre. Diese Legierungen waren jedoch in ihrer Korrosionsgeschwindigkeit stark gehemmt und entwickelten Wasserstoff in einem körperverträglichen Maße.

Weitere neuere Magnesiumlegierungen für resorbierbare Implantate enthalten Seltene Erden, vorzugsweise neben Lithium. Mit diesen Legierungen wird die Resorption des Implantats bereits deutlich verzögert, es kommt jedoch auch mit diesen Materialien immer noch zu sichtbarer Wasserstoffentwicklung und Gastaschen im Gewebe. Für viele Anwendungen ist die Korrosionsrate der seltenerdenhaltigen Legierungen noch zu hoch, da die mit der Resorption verbundenen Festigkeitsverluste im Heilungs- oder Gewebenachbildungsprozess zu früh eintreten.

Es besteht daher ein Bedürfnis an resorbierbaren Magnesiumwerkstoffen für medizinische Implantate mit gegenüber den bisher bekannten Werkstoffen verzögerter Resorption. Besonders vorteilhaft wäre es, wenn die Resorption an Einsatzort und Einsatzzweck angepasst werden könnte.

Magnesiumimplantate besitzen gegenüber Stahl- und Titanimplantaten außerdem große Vorteile bezüglich ihrer mechanischen Eigenschaften, insbesondere ihrer Festigkeit. Bei direkter Verbindung des Implantats mit menschlichem Knochen ist eine "angepasste Festigkeit" des Implantatwerkstoffs an die Knochenfestigkeit erwünscht. Gerade Magnesiumlegierungen besitzen im Vergleich mit Knochen ähnliche E-Module, so dass man der angestrebten Isoelastizität zwischen Implantat und Knochen näher kommen kann.

Es besteht daher auch ein hohes Bedürfnis für vollständig korrosionsresistente Magnesiumwerkstoffe, die für Dauerimplantate, Prothesen und mit Körperflüssigkeit und/oder Körpergeweben in Kontakt kommende medizinische Instrumente und Geräte eingesetzt werden könnten.

Die Aufgabe der Erfindung besteht daher darin, Magnesiumwerkstoffe für medizinische Implantate, Prothesen bzw. Protheseteile und medizinische Instrumente und Geräte, insbesondere OP-Bestecke und -Werkzeuge, zu finden, deren Korrosionswiderstand bis hin zur vollständigen Korrosionsfestigkeit einstellbar ist.

Diese Aufgabe wird durch die Verwendung eines durch Modifikation mit Halogeniden in seiner Korrosivität veränderten Magnesiumwerkstoffs für die Herstellung medizinischer Implantate, Protheseteile, Prothesen und medizinischer Instrumente, Geräte und Hilfsmittel gelöst, wie in den Ansprüchen ausgeführt.

Die bisher bekannten Magnesiumwerkstoffe sind schnell oder mittelfristig resorbierbar. Durch Modifikation mit Halogeniden wird der Korrosionswiderstand erhöht, und zwar umso mehr je höher die Halogenidionen-Konzentration in dem Werkstoff insgesamt oder der Werkstückoberfläche ist. Der Korrosionswiderstand kann so angepasst werden, dass die für den Einsatzzweck gewünschte Resorption eingestellt werden kann. Ab einem Fluoridionenanteil von ca. 10 bis 12 at% kann der Werkstoff oder der mit dieser Konzentration beaufschlagte Bereich als über die Lebensdauer des Implantats, der Prothese oder des medizinischen Instruments oder Geräts vollständig korrosionsfest angesehen werden. Auf diese Weise können auch für Dauerimplantate, Prothesen, Prothesenteile, Instrumente, Geräte usw. die vorteilhaften mechanischen Eigenschaften von Magnesiumwerkstoffen genutzt werden.

Für medizinische Geräte, insbesondere chirurgische Instrumente und Geräte wie z.B. OP-Bestecke, -Werkzeuge und -Geräte weist der hier verwendete halogenidmodifizierte Magnesiumwerkstoff ebenfalls beträchtliche Vorteile auf, denn er ist nicht magnetisch so dass darauf hergestellte Instrumente und Geräte u.a. für Operationen im Wirkungsbereich eines Kernspintomographen verwendet werden können. Der Werkstoff stellt sich gleichzeitig gut im Röntgenbild dar und ist daher auch beim Einsatz des Computertomographen gut geeignet.

Die Modifikation der als solches bekannten Magnesiumwerkstoffe erfolgt gemäß dieser Erfindung durch das Zulegieren von Halogen mit Hilfe von salzförmigen Halogenverbindungen, nämlich von Halogeniden. Das Einbringen der Halogenide und damit der Halogenidionen in den Werkstoff kann im Zuge üblicher Legierungsverfahren erfolgen.

Dabei werden Fluoride verwendet.

Als Fluoride werden solche Metallfluoride und komplexen Metallfluoride verwendet, die thermodynamisch instabiler als MgF₂, CaF₂ und LiF sind, so dass sich im Verlauf der Legierungsbildung MgF₂, CaF₂ und LiF bilden können, d. h. es werden AlF₃, KBF₄ und Na₃AlF₆ verwendet. Die Konzentration der Fluoride in dem Magnesiumwerkstoff wird vorzugsweise auf 1 bis 15 at% F, weiter vorzugsweise auf 1,5 bis 2,5 at% eingestellt.

Die Salze können Ober verschiedene Routen in den Magnesiumwerkstoff eingebracht werden, so z. B. mittels Gaslegierens, schmelzflüssigen Legierens, mechanischen Legierens, Schleuderguss, Reaktionsmahlen sowie Diffusionslegieren, die hier stellvertretend für weitere Techniken genannt werden. Der halogenmodifizierte Werkstoff kann wie für Magnesiumwerkstoffe bekannt zusätzlich behandelt werden, beispielsweise durch thermomechanische Verfahren, durch sequentielles Strangpressen, Homogenisieren und Auslagern. Das Metall kann spanend oder formgebend bearbeitet werden, z. B. durch Walzen, Drehen, Schmieden oder Stanzen.

Beim Diffusionslegieren werden Halbzeuge über eine bestimmte Zeit unter erhöhtem Druck und erhöhter Temperatur behandelt. Vorzugsweise werden Halbzeuge aus einem Magnesiumwerkstoff, d. h. aus Reinmagnesium oder einer Magnesiumbasislegierung, in ein Halogenid, vorzugsweise Aluminiumfluorid (AlF₃), eingebettet und bei Temperaturen von bis zu 850 °C, vorzugsweise um 420 °C, z. B. über 24 Stunden diffusionslegiert. Zu den Diffusionslegierungsverfahren gehört das Pulvereinpackverfahren. Wie mit Hilfe von Tauchversuchen in aggressivem, sythetischem Meerwasser nachgewiesen werden konnte, werden durch das Diffusionslegieren korrosionsstabile Deckschichten ausgebildet, die im pH-Intervall zwischen 3 und 14 schützen.

Das Schmelzlegieren wird erfindungsgemäß mit einem Zuschlag von Halogeniden zur Metallschmelze durchgeführt, vorzugsweise unter Zusatz von 1 bis 15 at% F, weiter vorzugsweise 1,5 bis 2,5 at% F. In bevorzugter Ausführung wird AlF₃ verwendet.

In Weiterbildung der Erfindung kann der mit Halogenid modifizierte Magnesiumwerkstoff auf die Metalloberfläche eines Werkstücks aufgebracht werden, insbesondere durch Aufdampfen oder durch Metallspritz- oder Sintertechniken auf vorgefertigte Implantate, Prothesen und medizinische Instrumente und Geräte. U.a. geeignet sind CVD- oder PVD-Verfahren, thermisches Spritzen im Lichtbogen oder Plasma sowie das Co-Strangpressen.

Als Magnesiumwerkstoff wird im Rahmen dieser Erfindung reines Magnesium oder eine Magnesiumlegierung, die Anteile von Lithium und/oder Calzium und/oder Aluminium und/oder Seltenen Erden enthält, verwendet.

Sowohl Magnesium als auch Calzium und Lithium bilden stabile Halogenide aus, die an der Oberfläche eines Werkstücks eine gegen Korrosion schützende Deckschicht bilden können. Beispielsweise entstehen bei Verwendung des derzeit bevorzugten AlF₃ als Prozesssalz während des Legierens die thermodynamisch stabileren Salze MgF₂, CaF₂ und LiF

Bevorzugt enthält der Magnesiumwerkstoff Lithium in einem Anteil von 0 bis 7 mas%, Aluminium in einem Anteil von 0 bis 16 mas%, Calzium in einem Anteil von 0 bis 5 mas% und Seltene Erden, vorzugsweise Cer und/oder Neodym und/oder praseodym, in einem Anteil von 0 bis 8 mas% und Yttrium in einem Anteil von O bis 7 mas%.

Als halogenmodifizierbare Basiswerkstoffe kommen insbesondere LAE 442 (MgLi4Al4SE2 mas%), MgY4SE3Li2,4 mas%, MgLi12 at%, MgLi40 at%, MgCa3O at%, AZ31 und AZ91 in Betracht.

Die Seltene Erden enthaltenden Magnesium- und Magnesium-Lithium-Legierungen sind selbst bereits durch den Einfluss der Seltenen Erden korrosionsgehemmt und können durch zusätzliches Auflegieren mit Halogeniden, insbesondere Fluoriden noch korrosionsresistenter gemacht werden. Als solches schneller korrodierende Magnesium-Basiswerkstoffe benötigen einen höheren Anteil an Fluoridbeimengung um einen vergleichbaren Effekt zu erzielen. Durch geeignete Auswahl der Legierungspartner lassen sich praktisch korrosionsresistente Werkstoffe erhalten.

Die Erfindung umfasst medizinische Implantate, insbesondere Befestigungselemente für Knochen, beispielsweise Schrauben, Platten oder Nägel, Anker, Stifte, Pacer, Cages, Knöpfe, Cerclagen, chirurgisches Nahtmaterial, wie Fäden oder Drähte, Folien und Netze (u.a. für die Wund- oder Bruchversorgung), Wund-, Naht- und Darmklammern, Gefäßclips, Abrasivpartikel für Wasserstrahlschneiden, Prothesen im Bereich des Hart- und Weichgewebes, zugehörige Protheseteile und medizinische Instrumente und Geräte, insbesondere OP-Bestecke, -Werkzeuge und Geräte, die zumindest teilweise aus den vorstehend beschriebenen, mit Halogeniden modifizierten Magnesiumwerkstoffen bestehen, oder die wenigstens an Teilen ihrer Oberfläche mit halogeniden modifiziert sind. Bevorzugt ist eine Modifikation mit bis zu 15 at% F.

Die eingesetzten Fluormengen sind für den Stoffwechsel wegen der geringen Freisetzung unkritisch; bei den praktisch korrosionsstabilen Legierungen mit höherem Fluoranteil wird Fluor nur vernachlässigbar über längere Zeiträume freigesetzt.

Im folgenden wird ein Beispiel für einen erfindungsgemäß zu verwendenden halogenmodifizierten Magnesiumwerkstoff gegeben.

### Beispiel:

Basiswerkstoff LAE442 (MgLi4Al4SE2 mas%), durch schmelzflüssiges Legieren mit 2 at% AlF₃ im Tiegel auflegiert.

Die fluormodifizierte Legierung weist einen um Faktor 10 besseren Korrosionswiderstand in aggressiven Elektrolyten (synthetisches Meerwasser als Untersuchungsmedium, vergleichbare Erbgebnisse mit 5 %iger NaCl-Lösung) und bereits im Gusszustand befriedigende mechanische Kennwerte auf:
R_{p0,2} = 80 MPa
Rₘ = 180 MPa
A₅ = 8 %

## Patentansprüche

1. Medizinisches Implantat, Prothese, Prothesenteil, medizinisches Instrument, medizinisches Gerät oder medizinisches Hilfsmittel, das zumindest teilweise einen mit Halogeniden modifiziertem Magnesiumwerkstoff enthält, wobei dieser modifizierte Magnesiumwerkstoff erhältlich ist durch Zulegieren von Fluoriden mit Hilfe von salzförmigen Halogenverbindungen, **dadurch gekennzeichnet, dass** als Fluoride KBF₄, Na₃AlF₆ oder AlF₃ eingesetzt werden.

2. Medizinisches Implantat, Prothese, Prothesenteil, medizinisches Instrument, medizinisches Gerät oder medizinisches Hilfsmittel nach Anspruch 1, **dadurch gekennzeichnet, dass** die Konzentration der Fluoride in dem Magnesiumwerkstoff auf bis zu 15 at% F eingestellt wird.

3. Medizinisches Implantat, Prothese, Prothesenteil, medizinisches Instrument, medizinisches Gerät oder medizinisches Hilfsmittel nach Anspruch 2, **dadurch gekennzeichnet, dass** die Konzentration der Fluoride in dem Magnesiumwerkstoff auf 1,5 bis 2,5 at% F eingestellt wird.

4. Medizinisches Implantat, Prothese, Prothesenteil, medizinisches Instrument, medizinisches Gerät oder medizinisches Hilfsmittel nach einem der vorherigen Ansprüche, **dadurch gekennzeichnet, dass** die Halogenide durch Diffussionslegieren oder Schmelzlegieren zulegiert werden.

5. Medizinisches Implantat, Prothese, Prothesenteil, medizinisches Instrument, medizinisches Gerät oder medizinisches Hilfsmittel nach einem der vorherigen Ansprüche, **dadurch gekennzeichnet, dass** der mit Halogenid modifizierte Magnesiumwerkstoff auf die Metalloberfläche eines Werkstücks aufgebracht wird.

6. Medizinisches Implantat, Prothese, Prothesenteil, medizinisches Instrument, medizinisches Gerät oder medizinisches Hilfsmittel nach Anspruch 5, **dadurch gekennzeichnet, dass** der mit Halogenid modifizierte Magnesiumwerkstoff durch Aufdampfen oder durch Metallspritz- oder Sinter-5, **dadurch gekennzeichnet, dass** der mit Halogenid modifizierte Magnesiumwerkstoff durch Aufdampfen oder durch Metallspritz- oder Sintertechniken auf vorgefertigte Implantat, Prothese, Prothesenteil, medizinisches Instrument, medizinisches Gerät oder medizinisches Hilfsmittel auf die Metalloberfläche eines Werkstoffstücks aufgebracht wird.

7. Medizinisches Implantat, Prothese, Prothesenteil, medizinisches Instrument, medizinisches Gerät oder medizinisches Hilfsmittel nach Anspruch 1, **dadurch gekennzeichnet, dass** der Magnesiumwerkstoff reines Magnesium ist oder eine Magnesiumlegierung, die Anteile von Lithium und/oder Calcium und/oder Aluminium und/oder Seltenen Erden enthält.

8. Medizinisches Implantat, Prothese, Prothesenteil, medizinisches Instrument, medizinisches Gerät oder medizinisches Hilfsmittel nach Anspruch 1, **dadurch gekennzeichnet, dass** der Magnesiumwerkstoff Lithium in einem Anteil von 0 bis 7 mas%, Aluminium in einem Anteil von 0 bis 16 mas%, Calcium in einem Anteil von 0 bis 5 mas% und Seltene Erden in einem Anteil von 0 bis 8 mas% und Yttrium in einem Anteil von 0 bis 7 mas%, wobei wenigstens ein Legierungsbestandteil mit mindestens 0,1 at% enthalten ist.

9. Medizinisches Implantat, Prothese, Prothesenteil, medizinisches Instrument, medizinisches Gerät oder medizinisches Hilfsmittel nach einem der vorherigen Ansprüche, **dadurch gekennzeichnet, dass** der mit Fluoriden modifizierte Magnesium-Basiswerkstoff LAE 442 (MgLi4Al4SE2) mas%), MgY4SE3Li2,4 mas%, MgLi12 at%, MgLi40 at%, MgCa30 at% ist.

## Claims

1. A medical implant, prosthesis, prosthesic part, medical instrument, medical device or medical auxiliary which contains at least in part a halid-modified magnesium substance, whereby said modified magnesium substance is obtainable by alloying of fluorides with the aid of halogen compound salts, **characterized in that** KBF₄, Na₃AlF₆ or AlF₃ are used as fluorides.

2. The medical implant, prosthesis, prosthetic part, medical instrument, medical device or medical auxiliary according to claim 1, **characterized in that** the concentration of the fluoride in the magnesium substance is up to 15 at% F.

3. Medical implant, prosthesis, prosthetic part, medical instrument, medical device or medical auxiliary according to claim 2, **characterized in that** the concentration of the fluoride in the magnesium substance is set at 1,5 to 2,5 at% F.

4. Medical implant, prosthesis, prosthetic part, medical instrument, medical device or medical auxiliary according to anyone of the proceeding claims, **characterized in that** the halogenides are alloyed by diffusion alloying or melt alloying.

5. Medical implant, prosthesis, prosthetic part, medical instrument, medical device or medical auxiliary according to anyone of the proceeding claims, **characterized in that** the halid-modified magnesium substance is applied to the metal surface of a work piece.

6. Medical implant, prosthesis, prosthetic part, medical instrument, medical device or medical auxiliary according to claim 5, **characterized in that** the halid-modified magnesium substance is applied to the metal surface of a work piece by vapour deposition or by metal spraying or sintering techniques onto prefabricated implants, prosthesis, prosthetic part, medical instrument, medical device or medical auxiliary.

7. Medical implant, prosthesis, prosthetic part, medical instrument, medical device or medical auxiliary according to claim 1, **characterized in that** the magnetic substance is pure magnesium or a magnesium alloy which contains proportions of lithium and/or calcium and/or aluminium and/or rare earth metal.

8. Medical implant, prosthesis, prosthetic part, medical instrument, medical device or medical auxiliary according to claim 1, **characterized in that** the magnesium substance contains lithium in a proportion of 0 to 7 mas%, aluminium in a proportion of 0 to 16 mas%, calcium in a proportion of 0 to 5 mas% and rare earth metal in a proportion of 0 to 8 mas% and yttrium in a proportion of 0 to 7 mas%, whereby at least one alloy component with at least 0.1 at% is contained.

9. Medical implant, prosthesis, prosthetic part, medical instrument, medical device or medical auxiliary according to anyone of the proceeding claims, **characterized in that** the fluoride modified magnesium base substance is LAE 442 (MgLi4Al4SE2) mas%, MgY4SE3Li2,4 mas%, MgLi12 at%, MgLi40 at%, MgCa30 at%.

## Revendications

1. Implant médical, prothèse, partie de prothèse, instrument médical, appareil médical ou accessoire médical dont au moins une partie contient un matériau à base de magnésium modifié par halogénures, ce matériau à base de magnésium modifié pouvant être obtenu en alliant des fluorures au moyen de composés halogénés sous forme de sels,
**caractérisé en ce que**
l'on utilise comme fluorures KBF₄, Na₃AlF₆ ou AlF₃.

2. Implant médical, prothèse, partie de prothèse, instrument médical, appareil médical ou accessoire médical selon la revendication 1, **caractérisé en ce que** la concentration des fluorures dans le matériau à base de magnésium est réglée jusqu'à 15 % atomiques de F.

3. Implant médical, prothèse, partie de prothèse, instrument médical, appareil médical ou accessoire médical selon la revendication 2, **caractérisé en ce que** la concentration des fluorures dans le matériau à base de magnésium est réglée entre 1,5 et 2,5 % atomiques de F.

4. Implant médical, prothèse, partie de prothèse, instrument médical, appareil médical ou accessoire médical selon l'une des revendications précédentes, **caractérisé en ce que** les halogénures sont alliés par alliage par diffusion ou alliage par fusion.

5. Implant médical, prothèse, partie de prothèse, instrument médical, appareil médical ou accessoire médical selon l'une des revendications précédentes, **caractérisé en ce que** le matériau à base de magnésium modifié par halogénure est appliqué sur la surface métallique d'une pièce.

6. Implant médical, prothèse, partie de prothèse, instrument médical, appareil médical ou accessoire médical selon la revendication 5, **caractérisé en ce que** le matériau à base de magnésium modifié par halogénure est appliqué par vaporisation ou par des techniques de pulvérisation de métal ou de frittage sur la surface métallique d'une pièce en matériau de l'implant médical, prothèse, partie de prothèse, instrument médical, appareil médical ou accessoire médical préfabriqué.

7. Implant médical, prothèse, partie de prothèse, instrument médical, appareil médical ou accessoire médical selon la revendication 1, **caractérisé en ce que** le matériau à base de magnésium est le magnésium pur ou un alliage de magnésium qui contient une proportion de lithium, de calcium, d'aluminium et/ou de terres rares.

8. Implant médical, prothèse, partie de prothèse, instrument médical, appareil médical ou accessoire médical selon la revendication 1, **caractérisé en ce que** le matériau à base de magnésium contient du lithium en proportion de 0 à 7 % en masse, de l'aluminium en proportion de 0 à 16 % en masse, du calcium en proportion de 0 à 5 % en masse, des terres rares dans une proportion de 0 à 8 % en masse et de l'yttrium en proportion de 0 à 7 % en masse, au moins un composant d'alliage étant présent à au moins 0,1 % atomique.

9. Implant médical, prothèse, partie de prothèse, instrument médical, appareil médical ou accessoire médical selon l'une des revendications précédentes, **caractérisé en ce que** le matériau de base en magnésium modifié par fluorures est le LAE 442 (MgLi4Al4SE2) % en masse, MgY4SE3Li2,4 % en masse, MgLi12 % atomiques, MgLi40 % atomiques ou MgCa30 % atomiques.
